# EUROPEAN PATENT APPLICATION

(11) **EP 3 093 028 A2**
(43) Date of publication of application: **16.11.2016**
(21) Application number: 16164949.6
(22) Date of filing: 12.04.2016
(51) Int. Cl.: A61K 47/48, A61K 38/18, A61K 31/7105, A61P 9/00

(54) **FORMULATIONS FOR INHIBITION FOR PCSK9 FOR THE TREATMENT OF HYPERCHOLESTEROLEMIA**

(30) Priority: 20.04.2015 GB 201506654
(71) Applicant: Heart Biotech Limited, London WS1 1YN (GB)
(72) Inventor: YACOUB, Magdi Habib, London, W1S 1YN (GB); EL SHERBINY, Ibrahim, 12588 Giza (EG)
(74) Representative: Lind, Robert

(57) **Abstract**

Described herein are pharmaceutical formulations for the treatment of hypercholesterolemia, comprising nanoparticles having a polymeric hydrophobic core which is associated with an inhibitor of PCSK9. The inhibitor may be EGF-A, EGF-B, or an siRNA. Preferred polymers include chitosan. The nanoparticle may further include a hydrophilic shell coating the core.

## Description

### FIELD OF THE INVENTION

The present invention relates to pharmaceutical formulations for the treatment of hypercholesterolemia. Aspects of the invention relate to formulations suitable for inhalable, injectable or oral administration. In preferred embodiments, the formulations make use of nanoparticles.

### BACKGROUND TO THE INVENTION

### Hyperlipidemia:

High level of low density lipoproteins (LDL) is a major risk factor for cardiovascular diseases. It is estimated that reducing LDL in blood by 39 mg/dL, cardiovascular disorders are reduced by 22%.¹ The main choice for reduction of LDL in high risk patients is the use of statins which act by inhibition of HMG-coAreductase, a central enzyme in the synthesis of cholesterol. However, the use of HMG-coAfails to reach the targeted blood level of LDL 1.8 mmol/L in 70% of patients after 6 months of treatment indicating the need for novel LDL lowering agents.

### PCSK9 structure:

PCSK9, initially calledneural apoptosis-regulated convertase 1(NARC-1), is a serine protease composed of 692 amino acids and a member of proproteinconvertase family. The mature enzyme is around 60 kDa. Secreted PCSK9 may exist as monomer, dimer or trimer in the circulation. The LDL receptors degrading activity of PCSK9 is directly correlated to the levels of dimeric and trimeric form of the enzyme.²PCSK9 has a catalytic N-terminal prodomain, substilin-like catalytic domain and a C-terminal domain. It is found mainly in the liver, kidney, small intestine and central nervous system.³The PCSK9 must undergo self-cleavage between the prodomain and the catalytic for activation of the enzyme. PCSK9 differs from other members of the convertase family in not requiring a second activation cleavage within the prodomain. As predicted, PCSK9 contains a classical serine protease catalytic triad with Ser386, His226 and Asp186 that can be overlayed with other related enzymes as proteinase K, furin and substilisin E. The C terminus of the prodomain, Gln152, forms hydrogen bonds with His226 and occupies the oxyanion hole found between the Ser386 backbone nitrogen and the Asn317 side chain amide.⁴

### Physiology:

There are controversial data regarding other physiological roles of PCSK9 in the body. PCSK9 is expressed by pancreatic δ cells. Some studies indicates that secreted PCSK9 is not related to insulin secretion ⁵ while another study done by Mbikay et al. showed that PCSK-9 mice suffer from impaired glucose tolerance and pancreatic islets abnormalities. ⁶

Proproteinconvertasesubtilisin/kexin type 9 (PCSK9) has been proposed as a target for treatment of hypercholesterolemia. PCSK9 is a serine protease enzyme that binds to LDL receptors leading to their degradation and subsequent increase in circulating LDL.Approximately 40% of circulating PCSK9 is found bound to LDL reducing its binding affinity to LDL receptors.⁷ The reaction between PCSK9 and LDL receptors occurs in the ratio 1:1 and with a *K*_{d} value ranging from 170 and 840 nM.⁸⁴. PCSK9 induces LDL receptor degradation in the hepatocytes, macrophages and fibroblast but not in the kidney or adrenal glands. In normal conditions, the enzyme is completely inactive in the central nervous system; however it is activated during development or after ischemic stroke. The function of this tissue-specific activity of the enzyme needs more investigation.⁹¹⁰

PCSK9 is mainly regulated by the level of intracellular cholesterol through modulating the translocation of sterol-responsive element-binding protein 2 which coordinates several factors affecting the synthesis and uptake of cholesterol including HMG-CoA synthase, HMG-CoA reductase and LDL receptors.¹¹ Cholesterol down-regulation of PCSK9 can also be mediated through the histone acetyltransferase cofactor.

### PCSK9 related disorders:

Mutations that lead to loss of PCSK9 result in reduction of LDL and cardiovascular risk.¹²A study done by Arsenault et al. indicated that the effects of reduced PCSK9 activity may not be limited to LDL levels only but it is also linked to reduced VLDL and increased beneficial HDL.¹³It has also been proposed that statins therapy is associated with up-regulation of PCSK9 which limits the efficacy of statins. It was thus assumed that inhibition of PCSK9 will lead to increased LDL receptors and decreased blood cholesterol. The activity of PCSK9 is normally regulated by plasma LDL levels where LDL binds to the amino acids 31 - 52 of the enzyme reducing its binding affinity to LDL receptors.¹⁴

Moreover, PCSK9 was found to be elevated in HIV+ patients which can be associated to the hyperlipidemia that was previously considered a side effect of anti-HIV drugs. It also indicates that PCSK9 inhibitors may be particularly useful in the treatment of statin resistance in HIV+ patients. ¹⁵PCSK9 inhibitors are also assumed to be useful in the treatment of sepsis since LDL receptors play a major role in the clearance of pathogenic lipids that induce uncontrolled systemic inflammation and hyperthermia associated with sepsis. In a preclinical study, inhibition of PCSK9 in animal model of sepsis reduced the production of inflammatory cytokines, reduced hyperthermia and improved survival rate.¹⁶

The activity of PCSK9 is not limited to cholesterol metabolism. The enzyme is also involved in the regulation of cell apoptosis, proliferation and inflammation. PCSK9 up-regulation is involved in the apoptosis of neuronal cells during cerebral development. This proapoptotic activity is mediated through the degradation of ApoER2 protein.¹⁷ This apoptotic effect may explain the linkage between hyperlipidemia and peripheral vascular disorders as risk factors for the development of senile dementia and Alzheimer's disease.¹⁸ PCSK9 is also highly expressed in renal tissues and may have a role in maintaining Na⁺ balance and normal blood pressure. Renal PCSK9 is not able to degrade LDL receptors while it induces the degradation of Na⁺channels in the endoplasmic reticulum of the renal collecting duct. Modulation of PCSK9 activity may play a role in carcinogenesis. It was found that PCSK9 is up-regulated in cervical cancer, esophageal adenocarcinoma, and renalcarcinoma while it is down-regulated in breast and prostate cancer.

### Inhibitors of PCSK9:

1- The use of small interfering RNA (siRNA) to inhibit the synthesis of the protein. ALN-PCS was the first drug of this class to be tested in phase 1 clinical trials for treatment of hypercholesterolemia. No significant adverse effects were observed after a single dose of the drug. ALN-PCS provided prolonged and significant lowering of PCSK9 protein and subsequent decrease in serum LDL.¹⁹ Another study proposed the use of siRNA to target SREBP cleavage-activating protein (SCAP) which is an important protein in the regulation of PCSK9. In a preclinical trial performed on rhesus monkeys, a single dose of SCAP siRNA-LNP reagent resulted in up to 95% reduction of SCAP protein along with a maximum 66% reduction in PCSK9 protein which was achieved on day 16 after treatment.²⁰
   Some other methods utilizing nano systems have been developed for the delivery of siRNA including active and passive targeting methods:
   a) Stable nucleic acid-lipid particles: (SNALP): Cationic lipid bilayer liposomes of size around 100 nm used to encapsulate siRNA. The lipid bilayer consists of a mixture of fusogenic and cationic lipids allowing cellular uptake and endosomal release of loaded siRNA.
   b) Lipidoid: Over 1200 varieties of these synthetic lipid-like molecules have been developed and some of which have been utilized for the intracellular delivery of siRNA to silence various genes in the liver.
   c) Atelocollagen: biomaterial consisting of a fraction of pepsin-digested type I collagen of calfdermis.²¹
   d) Cholesterol-siRNA conjugate: Targeting of this conjugate system depends on the type of lipoprotein particles involved where HDL-cholesterol is taken up by adrenal glands, ovary, kidney and liver while LDL-cholesterol is targeted mainly to the liver. Another type of targeted conjugates is the dynamic polyconjugates which utilize binding to asialoglycoprotein receptors for targeting to hepatocytes.
   e) Cyclodextrin-containing polycation: This delivery system is more biocompatible than other cationic particles and may be modified with various molecules for targeting purposes.²²
2- Monoclonal antibodies against PCSK9 as Evolocumab to block the activity of PCSK9 and reduce LDL. It was found that the use of Evolocumab results in significant and consistent reduction in the enzyme activity and serum LDL regardless of the basal PCSK9 activity. ²³A 1 year clinical study involving 1359 using Evolocumab subcutaneous injections for treatment of Hypercholesterolemia showed prolonged safety and efficacy of the drug with a mean 52.3% reduction in LDL-C at the end of the study. ²⁴Another similar monoclonal antibody is alirocumab which is being developed by Regeron and Sanofi. In a 6 month clinical trial, alirocumab showed superior efficacy and similar safety compared to ezetimibe which acts by inhibition of cholesterol absorption from the intestine.²⁵ It has been observed that using a combination of mAbs targeting PCSK9 and statins yields better efficacy than either treatment alone.²⁶
3- Adnectin (BMS-962476) targeting PCSK9 have been developed for treatment of Hypercholesterolemia. Adnectins (also known as monobodies) are small proteins of around 10 kDa developed by Adnexus company with easily modifiable specificity targeting different molecules.
4- Small organic molecules are still being designed for the inhibition of PCSK9 using in silico drug design by Shifa Biomedical Corporation
5- Natural products have also been investigated for inhibition of PCSK9 activity. Curcumin, a polyphenolic phytochemical was found to be able to reduce the Serum LDL level through down regulation of PCSK9.²⁷

### BRIEF SUMMARY OF THE INVENTION

According to the present invention, there is provided a pharmaceutical composition comprising a nanoparticle, the nanoparticle comprising a polymeric hydrophobic core; and an inhibitor of PCSK9 associated with the core.

By "nanoparticle" is meant a composition having a mean particle size (preferably diameter) of less than 600 nm, preferably less than 500 nm. Preferably the mean diameter ranges from 1 to 500 nm, more preferably 10-250 nm. In preferred embodiments, the mean diameter is less than 250 nm, preferably less than 200 nm.

The mean particle size may be determined by any suitable method practiced in the art; examples of suitable methods are exemplified herein.

The inhibitor is preferably a peptide inhibitor, and most preferably is selected from EGF-A and EGF-AB. In alternative embodiments, the inhibitor may be a nucleic acid, preferably a siRNA. A preferred siRNA is ALN-PCS. The inhibitors may be associated with the core via covalent bonds to the polymer. The bonds may be such as, but not limited to, acid labile, amide, and acid labile hydrazine bonds. Alternatively (and preferably in the case where the inhibitor is a nucleic acid inhibitor), the inhibitor may be incorporated into the core without being bonded thereto.

The polymer making up the polymeric core may be a PEG polymer. The polymer may be monomethoxy poly(ethylene glycol)-block-poly(D,L-lactide), polyethylene glycol-*b-*polypropylene glycol-*b*-polyethylene glycol. Alternatively, the polymer may be poly (lactic acid), poly (lactic-co-glycolic acid), poly(ethylene glycol)-block-poly (lactic-co-glycolic acid), or poly (caprolactone) nanoparticles.

In certain embodiments, the polymer may be a chitosan or a chitosan-derivative. Suitable chitosan-derivatives include, but not limited to, oligochitosan-grafted-PEG, N-trimethyl chitosan, and/or chitosan derivatives having hydrophobic moieties such as oleic acid, phthaloyl, and butyl acrylate.Chitosan or chitosan derivatives are particularly preferred, due to chitosan's ability to enhance absorption in lung tissues through opening the intercellular tight junctions of the lung epithelium.

In preferred embodiments, the polymers are graft and block copolymers. Chitosan or chitosan derivatives are particularly preferred, due to chitosan's ease functionality and binding ability to PCSK9 inhibitor peptides.

The nanoparticle may further comprise a shell, coating the hydrophobic core. The shell may be hydrophilic, and may comprise polysorbate 80, polysorbate 20, or polysaccharides; these repel the plasma proteins and increase the polymeric nanoparticles'shelf-lives.

The shell may comprise crosslinked polymers. The shell can also make the formulation suitable for dry powder inhalation. The core carries the inhibitor, while also allowing for pulmonary delivery due to its size. The shell can help to make the physical form more suitable for one or another administration route, while also permitting sustained release of the nanoparticles. The crosslinked polymers are preferably cross-linked hydrogel polymers. These may be in the form of semi-interpenetrating polymeric networks (semi-IPNs) and full-IPNs. These semi- and full-IPNs are preferably based on natural polymers such as, but not limited to, chitosan and water soluble chitosan derivatives (such as carboxymethyl and PEGylated derivatives) in a combination with one or more of nontoxic, biocompatible, and biodegradable polymers including, but not limited to, hyaluronate, carrageenan and oligoguluronate.In some embodiments, only chitosan or chitosan derivatives are used. The polymers are crosslinked through any suitable method, including ionotropic gelation, polyelectrolyte complexation and/or H-bonding. The shell-core formulations may be produced using a spray-drying technique, spray gelation, or ionotropic gelation followed by lyophilization. Spray drying is preferred.

The shell is preferably swellable; more preferably the shell comprises a hydrogel and the hydrogel is swellable. This allows the hydrogel to absorb moisture from the lung or other delivery site and so permit release of the nanoparticles. The hydrogel is preferably able to swell to at least 200, 300, 400, 500% of the original (dry formulation) size. In a preferred embodiment, a shell of 2-5 µm dry diameter is able to swell to at least 20 µm diameter. The shell is preferably able to swell to the larger diameter within 10, 9, 8, 7, 6, 5, 4, 3, or 2 minutes from administration to the lungs of a patient. In a further preferred embodiment, the shell is able to swell to at least 10 times the dry size within 3 minutes from administration.

The hydrogel preferably comprises less than 10%, preferably less than 7.5%, more preferably less than 5, 4, 3, 2% water when in the dry formulation. In preferred embodiments, this is less than 2%.

The nanoparticles are preferably biodegradable, and are preferably biocompatible. By biodegradable is meant that the particles will break down naturally within the body under physiological conditions; preferably the conditions as found within the blood. By biocompatible is meant that the particles will not elicit an immune response from the patient.

The hydrophobic core may further comprise magnetically responsive particles; preferably paramagnetic particles, and most preferably super paramagnetic iron oxide nanoparticles (SPIONs). These particles may be conjugated to PEG.

The nanoparticles may further be conjugated to a label; for example, a fluorophore. This allows imaging and/or diagnostics to be carried out using the nanoparticles.

In embodiments wherein the inhibitor is a nucleic acid inhibitor, then the polymeric core is preferably cationic. The core may be self-assembled. Preferred polymers are cationic chitosan derivatives, including, but not limited to, PEG-grafted-medium molecular weight N-phythaloyl chitosan (PEG-g-NPhCs), PEG-grafted-medium molecular weight chitosan (PEG-g-MMWCs), and PEG-grafted-oligochitosan (PEG-grafted-OCs). These nano-systems allowed the formation of siRNA polyplexes, increased the stability of siRNA, improved cellular internalization, and showed low toxicity in the cell line A549 (Guzman-Villanueva D., EI-Sherbiny I.M, Vlassov A.V., et al. Int. J. Pharmaceutics 473 (2014), 579). Besides, the polyplexes obtained from the PEG-g-OCs system showed silencing activity in a GFP model in the cell line A549 as compared to the naked siRNA which did not show any silencing activity in the same cells.

The core may be pH responsive. This aspect of the invention may be suitable for intracellular release of the cargo, ALN-PCS. Smart pH-responsive nanoparticles for drug delivery are known, and are used in situations where it is desirable to release a bioactive or therapeutic agent or moiety from a carrier under certain pH conditions. Examples of the development and use of pH-responsive carriers are given in Stephanie J. Grainger and Mohamed E. H. EI-Sayed, "STIMULI-SENSITIVE PARTICLES FOR DRUG DELIVERY", in Biologically Responsive Hybrid Biomaterials, EsmaielJabbari and Ali Khademhosseini (Ed), Artech House, Boston, MA, USA, and in PS Stayton and AS Hoffman, "Smart pH-responsive carriers for intracellular delivery of biomolecular drugs", in V Torchilin (ed), Multifunctional Pharmaceutical Nanocarriers, Springer Science and Business Media, 2008.

The formulation may be suitable for inhalable, injectable and/or oral delivery.

The formulation is preferably for the treatment of hypercholesterolemia.

The polymeric nanoparticles may be produced via emulsion-solvent evaporation, polyelectrolyte complexation, ionotropic gelation, and/or self-assembly. In preferred embodiments, the nanoparticles may be produced via self-assembly following homogenization,stirring, or sonication of amphiphilic copolymer solutions.

In an alternative embodiment, the invention provides a pharmaceutical composition comprising a nanoparticle, the nanoparticle comprising a porous nanoparticle core; an inhibitor of PCSK9 associated with the core; and a polymeric shell coating the core.

Porous particles have been used as carriers for drugs, absorption and desorption of substances, pulmonary drug delivery, and tissue regeneration. Internal connected porosity plays an important role in determining the capacity, efficiency and release kinetics of the microspheres in hand. Kim et al (Kim HK, Chung HJ, Park TG. Biodegradable polymeric microspheres with "open/closed" pores for sustained release of human growth hormone. J Control Release. 2006; 112(2):167-174), for instance fabricated a pore-closing porous PLGA microsphere, loaded with recombinant human growth hormone (rhGH). For their controlled release, porous microspheres containing rhGH were treated with water-miscible solvents in an aqueous phase for the production of pore-closed micro¬spheres, and this process was performed in an ethanol vapor phase using a fluidized bed reactor. The results exhibited a high protein loading amount.

The inhibitor is preferably a peptide inhibitor, and most preferably is selected from EGF-A and EGF-AB.

The core is preferably a porous silica core. The core may be magnetic or paramagnetic. The core may be prepared using a modified sol-gel method. Other materials suitable for preparing a core include both organic and inorganic materials such as calcium carbonate (CaCO₃), hydroxyapatite, and biodegradable porous starch foam. These materials have merits like biocompatibility or have a higher porosity or can be easily modified.

The shell may be composed of pH-responsive poly(N-isopropylacrylamide-co-methacrylic acid). The shell may be fabricated through controlled polymerization precipitation in the presence of the prepared core. Alternative materials for the shell include chitosan or chitosan derivatives; or polymers such as polylactic acid, poly(lactic-co-glycolic acid) (PLGA), polycaprolactone (PCL), and copolymers of said polymers.

### BRIEF DESCRIPTION OF THE DRAWINGS

Figure 1. Multifunctional PEGylated magnetic biodegradable polymeric self-assembled nanoparticles functionalized with peptide inhibitor
Figure 2. PEG-grafted-medium molecular weight N-phythaloyl chitosan (PEG-g-NPhCs), PEG-grafted-medium molecular weight chitosan (PEG-g-MMWCs), and PEG-grafted-oligochitosan (PEG-grafted-OCs)
Figure 3. PEG-grafted-medium molecular weight chitosan-Oleic acid (PEG-g-CS-Oleic).
Figure 4. The developed siRNA-loaded polyplexes cationic nanoparticles based on PEG-g-NPHCs, PEG-g-OCs, and PEG-g- LMWCs.

### DETAILED DESCRIPTION OF THE INVENTION

The present invention provides novel formulations of therapeutics for the treatment of hypercholesterolemia. Preferred therapeutic agents include PCSK9-inhibitor peptides such as EGF-A and EGF-AB, and siRNA that inhibits PCSK9 synthesis. The agents are either bound to or incorporated into polymeric nanoparticles; in alternative embodiments, the agents may be associated with porous nanoparticles. Either form of nanoparticle may also be associated in certain embodiments with a polymeric shell.

Background art which may be of benefit in understanding the invention includes:

EI-Sherbiny IM, Abdel-Mogib M, Dawidar A, Elsayed A, and Smyth HDC.(2010) Biodegradable pH-responsive alginate-poly (lactic-co-glycolic acid) nano/micro hydrogel matrices for oral delivery of silymarin, CarbohydrPolym, 83, 1345-1354.

Guzman-Villanueva D., EI-Sherbiny IM, Vlassov AV, Herrera-Ruiz D. & Smyth HDC (2014). Enhanced cellular uptake and gene silencing activity of siRNA molecules mediated by chitosan-derivative nanocomplexes, Int J. Pharm 473 (2014) 579-590

EI-Sherbiny IM, and Smyth, HDC. (2010) Biodegradable nano-micro carrier systems for sustained pulmonary drug delivery: (I) self-assembled nanoparticles encapsulated in respirable/swellable semi-IPN microspheres. Int J. Pharm 395: 132-141.

EI-Sherbiny IM, and Smyth HDC. (2010) PLGA nanoparticles encapsulated in respirable/swellable hydrogel microspheres as potential carriers for sustained drug delivery to the lung. Annual Meeting of American Association of Pharmaceutical Scientists, New Orleans, LA.

Reference to these publications should not be taken as an admission that the contents of any particular document are relevant prior art. However, the skilled person is referred to each of these publications for details of ways in which nanoparticles may be produced.

Various preliminary studies (referred to in the citations listed above) performed with regard to options for injection, and oral therapy support the proposal that well designed polymeric nanoparticles carrier systems either bound to PCSK9 inhibitor peptides or loaded with siRNA will improve efficiency of PCSK9 inhibition, increase targeting, inhibit PCSK9 synthesis, reduce dose frequency, avoid immune system, and discharge easily from the body. Remarkably, the preliminary data has shown that:
(1) The loading of bioactive materials including drugs, siRNA, etc into polymeric nanoparticles has the potential to significantly enhance dissolution and absorption, and consequently can improve the bioavailability of the loaded cargo.
(2) the design and composition of the nanoparticlescan be modulated to allow them to bind to various PCSK9-inhibitor peptides, or encapsulate more bioactive molecules such as siRNA.
(3) biodegradation rates of the nanoparticles are controllable.
(4) the nanoparticlescan be used efficiently for injection or oral therapy.
(5) the polymeric nanoparticles decorated by PCSK9-inhibitor peptides, or encapsulating bioactive molecules such as siRNA can efficiently confer sustained efficacy.

### 1. Composition of Nanoparticles

Our preliminary studies showed that loading of hydrophobic active ingredients into nanoparticles (made of ubiquitous polymers such as PLGA) considerably enhances the dissolution/absorption of these ingredients. Building on these observations, a new series of specifically designed polymeric nanoparticles were prepared via self-assembly of a new series of amphiphilic block and graft copolymers. The synthesized block and graft copolymers are based on biodegradable, non-toxic, and biocompatible natural polymers and chemically modified natural polymers such as, but not limited to, alginate, carrageenan, cellulose derivatives, chitosan, and chitosan derivatives.

Materials: The amphiphilic copolymers were synthesized via chemical modifications of some natural polymers such as, but not limited to, chitosan and chitosan derivatives through introducing of hydrophilic long side chains (such as PEG) and/or hydrophobic short side chains moieties (mainly, oleic acid, cholanic acid, stearic acid, butyl acrylate, and/ or phthaloyl moieties). Chitosan, a cationic polymer prepared through alkaline deacetylation of natural chitin, has been chosen as one such preferred polymer for the preparation of the nanoparticles due to its several desirable properties including non-toxicity, biodegradability, biocompatibility, in addition to its cationic nature that facilitates its physical binding to anionic bioactive ingredients such as siRNA (see Parka JH, Kwon S, Lee M, Chung H, Kim JH, Kim YS, Park RW, Kim IS, Seo SB, Kwon IC, and Jeong SY. (2006) Self-assembled nanoparticles based on glycol chitosan bearing hydrophobic moieties as carriers for doxorubicin: in vivo biodistribution and anti-tumor activity. Biomaterials, 27: 119-126).

Preparation Method: The self-assembled polymeric nanoparticles were prepared with (and without) homogenization of different concentrations of the modified polymers solutionsfor different intervals. The effect of relative compositions plus the different preparation parameters onto the physicochemical characteristics (mainly particle size) of the resulting nanoparticles were investigated extensively, to ensure desired properties have been achieved.

Drug loading: The loaded nanoparticles of different architectures were prepared in the same manner used for plain nanoparticles then either bound to the PCSK9-inhibitor peptides, or in situ or post-loaded with the siRNA, ALN-PCS. The effect of relative compositions plus the different preparation parameters onto both bioactive ingredients loading capacity (%) of the produced nanoparticles were studied in detailto determine optimal loading.

### 2. Nanoparticle Characterization

Polymer Characterization: The prepared and chemically modified polymers used as pre-cursors for the polymersomes and nanoparticles were characterized using different analytical equipment such as FT-IR, elemental analysis, thermogravimetric analysis (TGA), and differential scanning calorimetry (DSC). Also, the crystallography patterns of modified polymers were examined using X-ray diffraction (XRD).

Physicochemical characterization of the developed nanoparticles: The physicochemical characteristics of the developed polymersomes and nanoparticles such as particle size, moisture content, and surface morphology were investigated using dynamic light scattering (DLS), moisture analyzer, scanning electron microscopy (SEM), and atomic force microscopy (AFM). Besides, the biodegradation rates of the polymersomes or nanoparticles were estimated. Both plain and bioactive moieties (bound PCSK9 inhibitor peptides, or siRNA)-loaded nanoparticles with optimum physicochemical characteristicswere selected for further investigation in vitro and in vivo.

### Examples

### (I) Synthesis of free- and inhibitor peptide-loaded polyethylene glycol-grafted-poly (lactic-co-glycolic acid) nanoparticles:

The therapeutic agents-free and loaded-polyethylene glycol-g-poly (lactic-co-glycolic acid)nanoparticles capped with carboxylic groups were prepared using "emulsion-solvent evaporation method" through a procedure similar to that described in our previous work (see EI-Sherbiny IM et al, 2010, CarbohydrPolym, 83, 1345-1354). Briefly, 0.5g of PEG-g-PLGA-COOH was dissolved in 30 ml of methylene dichloride. A 2% w/v aqueous poly(vinyl alcohol), PVA solution (50 ml) was prepared to which, the PEG-g-PLGA-COOH solution was added dropwise while sonication of the surfactant, PVA solution at about 40 Watt. The mixture was then sonicated for further 3 min at 45% amplitude to create an oil-in-water emulsion. The sonication process was repeated twice until the desired size of the nanoparticles was obtained. The sonication step was performed in an icebath with the aid of pulse function (10 s pulse on, and 5 s pulse off) in order to avoid the heat built-up of the PEG-g-PLGA-COOH solution during the sonication. Afterwards, the emulsion was immediately poured into 80 ml of an aqueous 0.2% w/v PVA solution with rapid stirring. The resulting PEG-g-PLGA-COOH nanoemulsion was stirred overnight in uncovered container to evaporate both methylene chloride and ethanol. The resulting PEG-PLGA-COOH nanoparticles were then freeze dried and linked chemically to the selected inhibitor peptide through using EDC. The obtained plain and peptide-linked PEG-g-PLGA-COOH nanoparticles showed dense, and integrated spherical shapes with particle radius of 283 ± 8 and 296 ± 25 nm, respectively as determined by DLS.

### (II) Synthesis of free- and therapeutic agents-loaded chitosan-based polymersomes and nanoparticles:

### (1) Preparation of PEG-grafted-Chitosan copolymer

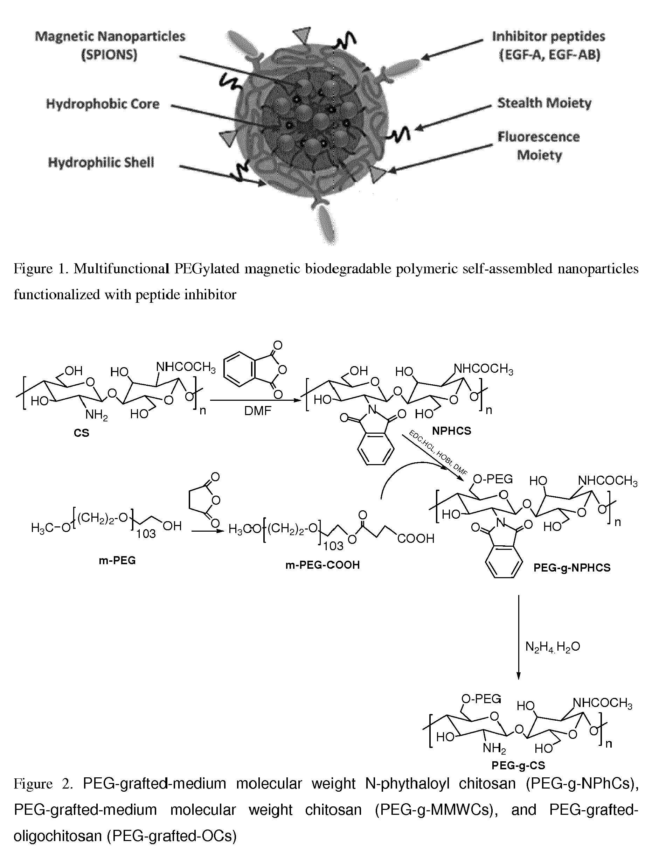

The PEG-grafted-chitosan was prepared (as illustrated in Figure 2) through a modified method of that reported in our previous study (EI-Sherbiny, I.M., & Smyth, H.D.C. (2012). Controlled release pulmonary administration of curcumin using swellable biocompatible nano-microparticles systems. Molecular Pharmaceutics, 9(2), 269-280). The method is described briefly as follows:
(i) Masking of the amino groups of Chitosan (CS): phthalic anhydride (44.8 g, 5 molequivalent to pyranose rings) was mixed with 10 g of CS in 150 ml DMF at 130°C in nitrogen atmosphere for 12 h. The resulting phthaloyl CS (PhCS) was collected through filtration after precipitation on ice-water, washed with methanol, and dried at 40°C under vacuum to produce the brown PhCS. (ii) Preparation of acid terminated PEG (PEG-COOH): methoxy-PEG (100 g, 20 mmol), triethylamine (2.02 g, 20 mmol), 4-dimethylaminopyridine, DMAP (2.44 g, 20 mmol), and succinic anhydride (2.4 g, 24 mmol) were mixed well in 200 ml dry dioxane. The mixture was stirred for 40 hrs under dry nitrogen atmosphere. Dioxane was then evaporated using a rotavap and the residue was taken up in carbon tetrachloride, filtered and precipitated by diethyl ether to produce the PEG-COOH. (iii) Conjugation of PhCS with PEG-COOH: PEG-COOH (37.9 g) was stirred with PhCS (5.0 g, 0.4 mol equivalent to PEG-COOH) in 60 ml DMF. Then, 1-hydroxybenzotrizole, HOBt (3.4 g, 3 mol equivalent to PEG-COOH) was added with stirring until a clear solution was obtained. The ethyl-3-(3-dimethylaminopropyl) carbodiimide-hydrochloride, EDC-HCl (4.25 g, 3 mol equivalent to PEG-COOH) was added with stirring the mixture overnight. A purified PEG-grafted-PhCS copolymer (5.47g, white product) was obtained after dialysis of reaction mixture against distilled water followed by extensive washing with ethanol. (iv) De-masking of the resulting PEG-graft-PhCS: PEG-g-PhCS (4 g) was heated up to 100°C with stirring under nitrogen atmosphere in 20 ml DMF. Then, 15 ml of hydrazine hydrate was added and the reaction was continued for 1 h. The resulting PEG-graft-CS was purified by dialysis against a (1:1) mixture of ethanol and deionized water then freeze dried.

### 2. Preparation of PEG-graft-CS-Oleic and PEG-graft-CS-Stearic acid copolymers:

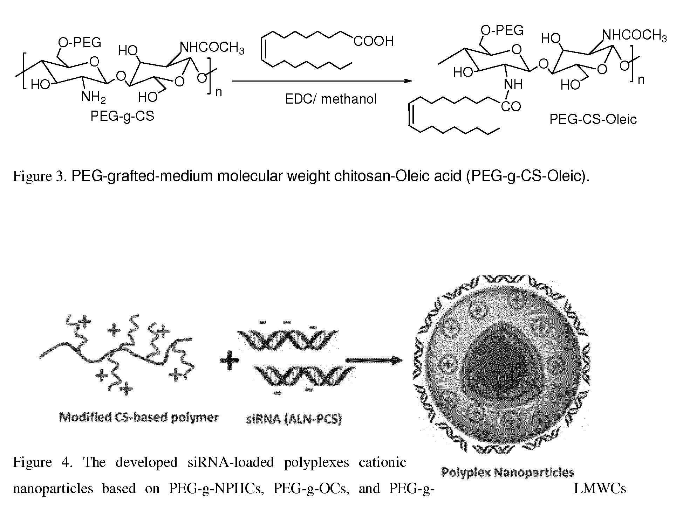

Hydrophobic side chains (HSC) including oleic acid, and stearic acid were coupled to CS-backbone of the PEG-graft-CSthrough formation of amide linkages through the EDC-mediated reaction as follows (Figure 3): In brief, 1 g PEG-graft-CS was dissolved in 0.5% (w/v) aqueous acetic acid solution (100 ml) and diluted with 85 ml methanol. HSC was then added to PEG-graft-CS solution at 0.4-0.5 mol/l glucosamine residue of CS followed by a drop-wise addition of 15 ml EDC methanol solution (0.08 g/l) while stirring. After 16 h, the reaction mixture was added to 250 ml of methanol/ammonia solution (7/3, v/v) with stirring. The resulting precipitate was filtered; washed with distilled water, methanol, and ether; and then dried under vacuum for 20 h at room temperature. The degree of substitution which represents the number of HSC groups per 100 amino groups of CS, was determined using normal titration.

### 3. Characterization of the modified polymers:

Both synthesized and the chemically modified polymers used as pre-cursors for the polymersomes and the self-assembled nanoparticles fabrication were characterized using various analytical techniques such as Fourier transform infrared (FT-IR), elemental analysis (EA), nuclear magnetic resonance (NMR), differential scanning calorimetry (DSC), and thermogravimetric analysis (TGA). Besides, the crystallography of modified polymers was investigated using X-ray diffraction (XRD).

### 4. FT-IR and elemental analysis data of some of the synthesized polymers and copolymers.

*PEG-COOH:* FT-IR (vₘₐₓ, cm⁻¹) 3502, 2879, 1743, 1114; (C₂₃₁H₄₆₁O₁₁₇), calculated (%): C 54.38, H 9.04; found (%), C 56.3, H 9.21. *PhCS:* FT-IR (vₘₐₓ, cm⁻¹) 3286, 2972, 1770, 1689, 1401, 1050, 727; (C₈H₁₃NO₅)_{0.2363}(C₆H₁₁NO₄)_{0.016}(C₁₄H₁₃NO₆)_{0.747}, calculated (%) (*DS* = 0.97) (%): C 55.71, H 4.86, N 5.21; found (%), C 60.27, H 4.80, N 4.97. *PEG-PhCS copolymer:* FT-IR (vₘₐₓ, cm⁻¹) 3411, 2901, 1739, 1712, 1091, 720, found EA (%), C 56.21, H 4.61, N 5.22. *PEG-CS copolymer:* FT-IR (vₘₐₓ, cm⁻¹) 3305, 2871, 1706, 1099; found EA (%), C 40.51, H4.74, N 14.09.

### 5. Development of free- and bioactive moieties (PCSK9-inhibitor peotides, or siRNA)-loaded modified CS-based self-assembled nanoparticles:

The resulting modified CS copolymers (PEG-graft-OCS, PEG-graft-MMWCS, PEG-graft-NPhOCS,PEG-graft-CS-Oleic, and PEG-graft-CS-Stearic) were used to develop a new series of self-assembled nanocarriers systems for inhibition of PCSK9. The self-assembled nanoparticles were prepared with and without sonication of different concentrations (0.02-1.5%) of the modified polymers solutions using a probe sonicator. The sonication step was carried out at different sonication powers (20-40 W) for different time intervals (20-120s). The effect of relative compositions in addition to the different preparation parameters on the physicochemical properties (particle size, surface morphology, inhibitor peptide loading/binding capacity, and moisture content) of the resulting nanoparticles was examined. The developed self-assembled nanoparticles showed particle radius of 83 ± 14 and 102 ± 8 nm for the free- and the peptide-loaded nanoparticles, respectively, as measured using DLS.

### Therapeutic use

Therapeutic-agent labelled nanoparticles prepared as described above, and linked to either peptide inhibitors (EGF-A or EGF-AB) or siRNA (ALN-PCS), may be tested for therapeutic efficacy in established in vitro and/or in vivo systems.

### References:

(1) Smith, S. C.; Benjamin, E. J.; Bonow, R. O.; Braun, L. T.; Creager, M. A.; Franklin, B. A.; Gibbons, R. J.; Grundy, S. M.; Hiratzka, L. F.; Jones, D. W.; et al. AHA/ACCF Secondary Prevention and Risk Reduction Therapy for Patients With Coronary and Other Atherosclerotic Vascular Disease: 2011 UpdateA Guideline From the American Heart Association and American College of Cardiology Foundation Endorsed by the World Heart Federation and the Preventive Cardiovascular Nurses Association. Journal of the American college of cardiology2011, 58, 2432-2446.
(2) Fan, D.; Yancey, P. G.; Qiu, S.; Ding, L.; Weeber, E. J.; Linton, M. F.; Fazio, S. Self-Association of Human PCSK9 Correlates with Its LDLR-Degrading Activity. Biochemistry2008, 47, 1631-1639.
(3) Norata, G. D.; Tibolla, G.; Catapano, A. L. Targeting PCSK9 for Hypercholesterolemia. Annual review of pharmacology and toxicology2014, 54, 273-293.
(4) Cunningham, D.; Danley, D. E.; Geoghegan, K. F.; Griffor, M. C.; Hawkins, J. L.; Subashi, T. A.; Varghese, A. H.; Ammirati, M. J.; Culp, J. S.; Hoth, L. R.; et al. Structural and Biophysical Studies of PCSK9 and Its Mutants Linked to Familial Hypercholesterolemia. Nature structural \& molecular biology2007, 14, 413-419.
(5) Langhi, C.; Le May, C.; Gmyr, V.; Vandewalle, B.; Kerr-Conte, J.; Krempf, M.; Pattou, F.; Costet, P.; Cariou, B. PCSK9 Is Expressed in Pancreatic $\delta$-Cells and Does Not Alter Insulin Secretion. Biochemical and biophysical research communications2009, 390, 1288-1293.
(6) Mbikay, M.; Sirois, F.; Mayne, J.; Wang, G.-S.; Chen, A.; Dewpura, T.; Prat, A.; Seidah, N. G.; Chretien, M.; Scott, F. W. PCSK9-Deficient Mice Exhibit Impaired Glucose Tolerance and Pancreatic Islet Abnormalities. FEBS letters2010, 584, 701-706.
(7) Kosenko, T.; Golder, M.; Leblond, G.; Weng, W.; Lagace, T. A. Low Density Lipoprotein Binds to ProproteinConvertaseSubtilisin/kexin Type-9 (PCSK9) in Human Plasma and Inhibits PCSK9-Mediated Low Density Lipoprotein Receptor Degradation. Journal of Biological Chemistry2013, 288, 8279-8288.
(8) Piper, D. E.; Jackson, S.; Liu, Q.; Romanow, W. G.; Shetterly, S.; Thibault, S. T.; Shan, B.; Walker, N. P. The Crystal Structure of PCSK9: A Regulator of Plasma LDL-Cholesterol. Structure2007, 15, 545-552.
(9) Ferri, N.; Tibolla, G.; Pirillo, A.; Cipollone, F.; Mezzetti, A.; Pacia, S.; Corsini, A.; Catapano, A. L. ProproteinConvertaseSubtilisinKexin Type 9 (PCSK9) Secreted by Cultured Smooth Muscle Cells Reduces Macrophages LDLR Levels. Atherosclerosis2012, 220, 381-386.
(10) Rousselet, E.; Marcinkiewicz, J.; Kriz, J.; Zhou, A.; Hatten, M. E.; Prat, A.; Seidah, N. G. PCSK9 Reduces the Protein Levels of the LDL Receptor in Mouse Brain during Development and after Ischemic Stroke. Journal of lipid research2011, 52, 1383-1391.
(11) Jeong, H. J.; Lee, H.-S.; Kim, K.-S.; Kim, Y.-K.; Yoon, D.; Park, S. W. Sterol-Dependent Regulation of ProproteinConvertaseSubtilisin/kexin Type 9 Expression by Sterol-Regulatory Element Binding Protein-2. Journal of lipid research 2008, 49, 399-409.
(12) Cohen, J. C.; Boerwinkle, E.; Mosley Jr, T. H.; Hobbs, H. H. Sequence Variations in PCSK9, Low LDL, and Protection against Coronary Heart Disease. New England Journal of Medicine2006, 354, 1264-1272.
(13) Arsenault, B. J.; Boekholdt, S. M.; Hovingh, G. K.; Kastelein, J. J.; Wareham, N. J.; Khaw, K.-T. Beyond LDL Cholesterol: Carriers of the PCSK9 R46L Variant Are Characterized by an Anti-Atherogenic Lipoprotein Profile Assessed by Nuclear Magnetic Resonance Spectroscopy. Circulation2014, 130, A11566-A11566.
(14) Tabas, I.; Williams, K. J.; Borén, J. Subendothelial Lipoprotein Retention as the Initiating Process in Atherosclerosis Update and Therapeutic Implications. Circulation2007, 116, 1832-1844.
(15) Kohli, P.; Ganz, P.; Ma, Y.; Scherzer, R.; Deeks, S. G.; Maka, K.; Wasserman, S. M.; Scott, R.; Hsue, P. PCSK9 Is Elevated in HIV+ Patients. Circulation2014, 130, A17751-A17751.
(16) Bray, N. Sepsis: PCSK9 Blockade Helps Clear Pathogenic Lipids. Nature Reviews Drug Discovery2014, 13, 886-887.
(17) Kysenius, K.; Muggalla, P.; Mätlik, K.; Arumäe, U.; Huttunen, H. J. PCSK9 Regulates Neuronal Apoptosis by Adjusting ApoER2 Levels and Signaling. Cellular and Molecular Life Sciences2012, 69, 1903-1916.
(18) Mahley, R. W.; Weisgraber, K. H.; Huang, Y. Apolipoprotein E4: A Causative Factor and Therapeutic Target in Neuropathology, Including Alzheimer's Disease. Proceedings of the National Academy of Sciences2006, 103, 5644-5651.
(19) Fitzgerald, K.; Frank-Kamenetsky, M.; Shulga-Morskaya, S.; Liebow, A.; Bettencourt, B. R.; Sutherland, J. E.; Hutabarat, R. M.; Clausen, V. A.; Karsten, V.; Cehelsky, J.; et al. Effect of an RNA Interference Drug on the Synthesis of ProproteinConvertaseSubtilisin/kexin Type 9 (PCSK9) and the Concentration of Serum LDL Cholesterol in Healthy Volunteers: A Randomised, Single-Blind, Placebo-Controlled, Phase 1 Trial. The Lancet2014, 383, 60-68.
(20) Jensen, K. K.; Tadin-Strapps, M.; Wang, S.-P.; Herath, K.; Hubert, J.; Kan, Y.; Rosa, R.; Szeto, D.; Williams, S.; Xie, D.; et al. PCSK9 and LDL-C Lowering Effect of SCAP Targeting siRNA in Mouse and Non-Human Primate Models. Circulation2014, 130, A18950-A18950.
(21) Minakuchi, Y.; Takeshita, F.; Kosaka, N.; Sasaki, H.; Yamamoto, Y.; Kouno, M.; Honma, K.; Nagahara, S.; Hanai, K.; Sano, A.; et al.Atelocollagen-Mediated Synthetic Small Interfering RNA Delivery for Effective Gene Silencing in Vitro and in Vivo. Nucleic acids research2004, 32, e109-e109.
(22) Peer, D.; Shimaoka, M. Systemic siRNA Delivery to Leukocyte-Implicated Diseases. Cell Cycle2009, 8, 853-859.
(23) Desai, N.; Giugliano, R.; Wasserman, S.; Gibbs, J.; Liu, T.; Scott, R.; Sabatine, M. Impact of Baseline PCSK9 Levels on the Efficacy of Evolocumab, a Monoclonal Antibody Against PCSK9. Circulation2014, 130, A16196-A16196.
(24) Koren, M. J.; Giugliano, R. P.; Raal, F. J.; Sullivan, D.; Bolognese, M.; Langslet, G.; Civeira, F.; Somaratne, R.; Nelson, P.; Liu, T.; et al. Efficacy and Safety of Longer-Term Administration of Evolocumab (AMG 145) in Patients With Hypercholesterolemia 52-Week Results From the Open-Label Study of Long-Term Evaluation Against LDL-C (OSLER) Randomized Trial. Circulation2014, 129, 234-243.
(25) Roth, E. M.; Taskinen, M.-R.; Ginsberg, H. N.; Kastelein, J. J.; Colhoun, H. M.; Robinson, J. G.; Merlet, L.; Pordy, R.; Baccara-Dinet, M. T. Monotherapy with the PCSK9 Inhibitor Alirocumab versus Ezetimibe in Patients with Hypercholesterolemia: Results of a 24week, Double-Blind, Randomized Phase 3 Trial. International journal of cardiology2014, 176, 55-61.
(26) Ni, Y. G.; Di Marco, S.; Condra, J. H.; Peterson, L. B.; Wang, W.; Wang, F.; Pandit, S.; Hammond, H. A.; Rosa, R.; Cummings, R. T.; et al. A PCSK9-Binding Antibody That Structurally Mimics the EGF (A) Domain of LDL-Receptor Reduces LDL Cholesterol in Vivo. Journal of lipid research2011, 52, 78-86.
(27) Tai, M.-H.; Chen, P.-K.; Chen, P.-Y.; Wu, M.-J.; Ho, C.-T.; Yen, J.-H. Curcumin Enhances Cell-Surface LDLR Level and Promotes LDL Uptake through Downregulation of PCSK9 Gene Expression in HepG2 Cells. Molecular nutrition \& food research2014, 58, 2133-2145.

## Claims

1. A pharmaceutical composition comprising a nanoparticle, the nanoparticle comprising a polymeric hydrophobic core; and an inhibitor of PCSK9 associated with the core.

2. The composition of claim 1, wherein the inhibitor is a peptide inhibitor, or is siRNA, and preferably wherein the inhibitor is selected from EGF-A and EGF-AB.

3. The composition of any preceding claim wherein the inhibitor is associated with the core via covalent bonds to the polymer, preferably wherein the covalent bonds are selected from acid labile, amide, and acid labile hydrazine bonds.

4. The composition of any preceding claim wherein the polymer making up the polymeric core is a PEG-based polymer; or
wherein the polymer is selected from monomethoxy poly(ethylene glycol)-block-poly(D,L-lactide), polyethylene glycol-*b*-polypropylene glycol-*b*-polyethylene glycol, poly (lactic acid), poly (lactic-co-glycolic acid), poly(ethylene glycol)-block-poly (lactic-co-glycolic acid), and poly (caprolactone); or wherein the polymer is a chitosan or a chitosan-derivative, and is preferably PEG-grafted-oligochitosan.

5. The composition of any preceding claim wherein the nanoparticle further comprises a shell, coating the hydrophobic core.

6. The composition of claim 5 wherein the shell is hydrophilic, optionally wherein the shell comprises crosslinked polymers, and wherein the crosslinked polymers are preferably cross-linked hydrogel polymers.

7. The composition of any of claims 5 or 6 wherein the shell is pH responsive.

8. The composition of any preceding claim wherein the hydrophobic core further comprises magnetically responsive particles.

9. The composition of any preceding claim wherein the nanoparticles are conjugated to a label.

10. The composition of any preceding claim wherein the core is pH responsive.

11. The composition of any preceding claim wherein the core is porous.

12. The composition of any preceding claim wherein the formulation is suitable for inhalable, injectable and/or oral delivery.

13. The composition of any preceding claim wherein the formulation is for the treatment of hypercholesterolemia.

14. A pharmaceutical composition comprising a nanoparticle, the nanoparticle comprising a porous nanoparticle core; an inhibitor of PCSK9 associated with the core; and a polymeric shell coating the core; preferably wherein the core is a porous silica or a porous polymeric core.

15. The composition of any preceding claim, further comprising a pharmaceutically acceptable carrier, diluent, and/or excipient.
